# EUROPEAN PATENT SPECIFICATION

(11) **EP 2 745 776 B1**
(45) Date of publication and mention of the grant of the patent: **10.04.2019**
(21) Application number: 13194968.7
(22) Date of filing: 28.11.2013
(51) Int. Cl.: A61B 5/097, A61M 16/04, B01D 46/02

(54) **Mouthpiece for breathing apparatuses and method to make said mouthpiece**
Mundstück für Atemvorrichtungen und Verfahren zur Herstellung des besagten Mundstücks
Embout buccal pour appareils respiratoires et procédé pour fabriquer ledit embout

(30) Priority: 29.11.2012 IT MI20122037
(43) Date of publication of application: 25.06.2014
(73) Proprietor: Lumed SRL, 20159 Milano (IT)
(72) Inventor: Santamarina, Fabio, 20144 Milano (IT)
(74) Representative: Petraz, Gilberto Luigi

(56) References cited:
- EP-A1- 2 098 166
- DE-U1-202012 007 148
- US-A- 5 655 526
- US-A- 6 010 458
- US-A1- 2010 041 062

## Description

### FIELD OF THE INVENTION

The present invention concerns a mouthpiece for breathing apparatuses, such as for example, but not only, spirometer machines, also commonly named spirometers. In particular, the mouthpiece allows to prevent the contamination of the spirometric instrument and the consequent crossover contamination between patients.

The present invention also concerns the method to make said mouthpiece.

### BACKGROUND OF THE INVENTION

It is known that in order to carry out diagnostic tests on lung functionality it is necessary to adopt measures which allow to prevent the contamination of the spirometric instrument through direct contact with the patient.

To this purpose it is necessary to associate with the instruments devices of a disposable type, also called mouthpieces, which allow to prevent the direct contact of the patient's mouth with the spirometric instrument.

In this field, mouthpieces made of ecological and biocompatible material are known, made of cardboard for example, consisting of a substantially cylindrical tube which is mounted on the instrument and which allows to distance the patient's mouth from the instrument, thus preventing direct contact. One example of this type of mouthpiece is described in the document EP-A-2.098.166.

These types of mouthpieces, even though widely used thanks to their low production cost and the ease with which they can be disposed of, have the disadvantage that they do not create a filtering barrier between the instrument itself and the patient, and thus do not eliminate the risk of crossover contamination between patients.

In fact, known mouthpieces made of cardboard, if the spirometric instrument is used for aspiration, does not prevent contaminating particles deposited on the instrument entering into contact with the patient.

To prevent crossover contamination between patients, mouthpieces for spirometric instruments are known, also disposable, consisting of a tube, or other device, made of plastic material and provided internally with a viral bacterial filter to filter the air sucked in and blown through the tube. In particular, the viral bacterial filter consists of a disc with sizes mating with those of the internal section of the plastic tube, which is attached in an intermediate position of the longitudinal extension and normally in a position substantially perpendicular to the direction of flow. An example of a filtering disc of this type is shown and described in the document DE-U-20.2012.007.148.

Mouthpieces consisting of a tube of plastic material, configured to be associated with a spirometric instrument, are also known, for example from the document US-A-6.010.458.

A sac-shaped filter to filter the air is attached to the tube made of plastic material with an end edge. The filter is attached to the tube in different ways, for example jointed.

These known mouthpieces made of plastic material are complex and costly to make, and are thus only used in environments in which the contamination between patients is particularly risky; therefore they do not cover all tests.

Moreover, mouthpieces made of plastic material have problems and high costs of disposal and are not very environmentally friendly for very widespread tests, also because of the amount of plastic material which is used, for example at least 20g in weight.

One purpose of the present invention is to make a mouthpiece for breathing apparatuses of the disposable type which is extremely simple to make and to dispose of.

Another purpose of the present invention is to make a mouthpiece for breathing apparatuses which allows to overcome the disadvantages of crossover contamination between patients.

Another purpose is to perfect a method to make a mouthpiece for breathing apparatuses which is simple and rapid.

The Applicant has devised, tested and embodied the present invention to overcome the shortcomings of the state of the art and to obtain these and other purposes and advantages.

### SUMMARY OF THE INVENTION

The present invention is set forth and characterized in the independent claims, while the dependent claims describe other characteristics of the invention or variants to the main inventive idea.

In accordance with the above purposes, a mouthpiece for breathing apparatuses, such as, merely by way of example, spirometer machines, comprises at least a tubular body provided with an internal surface defining a channel for the passage of the stream of air that is breathed out or in by a patient. The tubular body is configured to be installed on parts of the breathing apparatus to prevent direct contact of the apparatus and the patient.

The mouthpiece also comprises a filtering element of the sac type, hereafter called sac-shaped, attached peripherally with one open end edge to the internal surface of the tubular body and completely contained inside it, thus allowing to filter the air that is breathed out or in by the patient through the tubular body. The filtering element thus allows, with an extremely simple and economical product, easy to produce and to dispose of, to eliminate crossover contaminations between patients who use the same apparatus.

The tubular body is made of cardboard, and this allows to obtain a throwaway mouthpiece, easy to dispose of, limited in cost and lighter than the mouthpieces currently made of plastic material with integrated filters.

The tubular body has a uniform thickness along its longitudinal extension, and the filtering element is attached inside it without requiring particular attachment surfaces, ridges or other.

The tubular body has a first end with an oval shape to allow the patient to put it easily into his/her mouth.

The present invention also concerns the method to make a mouthpiece of the type described above, which provides at least a step of making a tubular body of cardboard, a step of making a sac-shaped filtering element and a step of attaching peripherally an open end edge of the filtering element to the internal surface of the tubular body.

### BRIEF DESCRIPTION OF THE DRAWINGS

These and other characteristics of the present invention will become apparent from the following description of one form of embodiment, given as a non-restrictive example with reference to the attached drawings wherein:
- fig. 1 is a perspective view of the mouthpiece for breathing apparatuses according to the present invention;
- fig. 2 shows a longitudinal section of the mouthpiece in fig. 1;
- fig. 3 shows a longitudinal section from III to III in fig. 2;
- fig. 4 is a schematic representation of an apparatus for making a mouthpiece;
- fig. 5 is a perspective view of part of the apparatus in fig. 4 in a first operating configuration;
- fig. 6 is a perspective view of part of the apparatus in fig. 4 in a second operating configuration.

To facilitate comprehension, the same reference numbers have been used, where possible, to identify identical common elements in the drawings.

### DETAILED DESCRIPTION OF ONE FORM OF EMBODIMENT

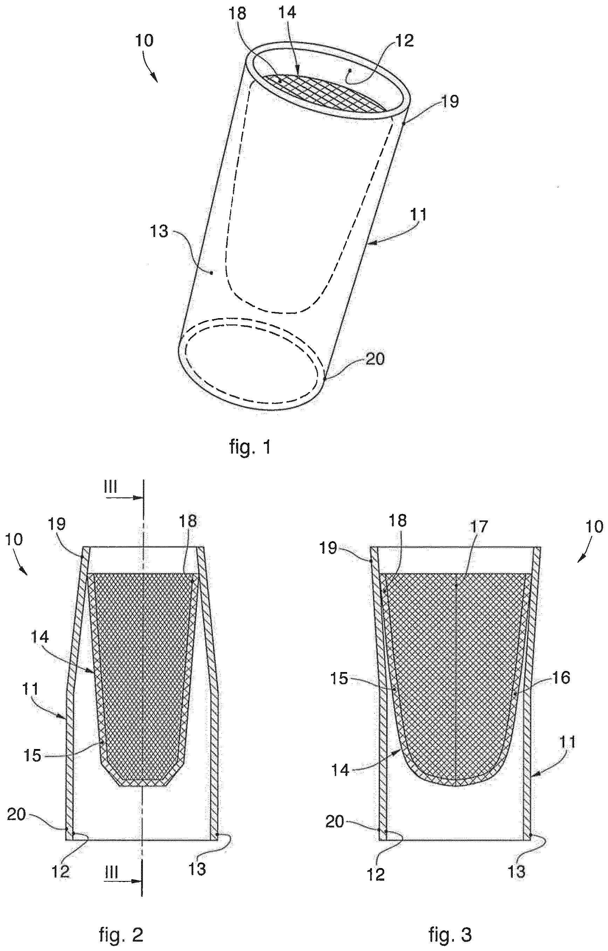

With reference to figs. 1-3, a mouthpiece for breathing apparatuses is indicated in its entirety by the reference number 10, and comprises a tubular body 11, or tube, provided with an internal surface 12 and an external surface 13.

The mouthpiece 10 is the disposable type and is configured to be associated with instruments of breathing apparatuses such as, merely by way of example, spirometric machines.

The tube 11 is preferably made of cardboard, and is therefore biocompatible and easy to dispose of.

A sac-shaped filtering element 14 is attached in the internal surface 12 of the tube 11, and comprises at least a filtering fabric, in this case two filtering fabrics 15 and 16 coupled with each other along a joining line 17 (fig. 3).

The tube 11 has a substantially uniform thickness along its entire longitudinal extension and, merely by way of example, is comprised between 0.5 mm and 1 mm.

The filtering element 14 has a substantially longitudinal development and is completely contained inside the tube 11, in its axial development.

The filtering fabrics 15 and 16 consist of a plurality of layers of filaments to define a thick filtering mesh, such as to ensure an adequate filtering power comparable to that of the bacterial filters described above and such as to respect the appropriate regulations. By way of example, the filtering fabrics 15 and 16 have a filtering capacity near to 99.99% of particulate with sizes greater than 1 µm.

The filtering capacity of the filtering fabrics 15 and 16 is proportional to the surface through which the stream of air exhaled by the patient passes. The resistance to the stream of air exhaled, put up by the filtering fabrics 15 and 16, increases as the filtering capacity increases, and therefore the thicker the meshes are, the greater the resistance encountered by the stream of air exhaled by the patient.

Merely by way of example, and to meet some of the respective regulations, the resistance to the stream of air exhaled is less than 1.5 cm/H₂O/l/s with a flow rate of 14 l/s.

In some forms of embodiment it is provided that the meshes that make up the filtering fabrics 15 and 16 are made of a material chosen at least from modacrylate, polypropylene or a combination thereof.

The sac-shaped filtering element 14 (figs. 1-3) has an open end edge 18 attached permanently and peripherally to the internal surface 12 of the tube 11 in proximity to a first end 19 of the latter.

By the term "attached permanently" we mean that the filtering element 14 cannot be detached from the tube 11 except by at least partly disintegrating either the tube 11 or the filtering element 14, thus rendering them in practice unusable. This guarantees that parts of the same mouthpiece cannot be used several times for different analyses.

The end edge 18 of the filtering element 14 is connected to the tube 11 by heat action, gluing, ultrasound or a combination thereof.

If a connection is provided by heat action, a circumferential portion of the end edge 18 is partly melted in order to make it solid with the internal surface 12 of the tube 11.

In particular, the end edge 18 of the filtering element 14 has a peripheral development substantially equal to the peripheral development of the internal surface 12 of the tube 11. In this way, when attached to the tube 11, the filtering element 14 occupies the entire cross section thereof.

The filtering element 14 is disposed with its convex portion facing toward a second end 20 of the tube 11, opposite the first end 19.

In some forms of embodiment it may be provided that the filtering element 14 extends axially into the tube 11 for at least 30% of its axial development.

The first end 19 of the tube 11 has an oval or elliptical shape, to confer upon it an anatomical shape so that the patient can easily put it in his/her mouth.

In the form of embodiment in figs. 2 and 3, the first end 19 has a ratio between its minimum and maximum size comprised between 0.90 and 0.70, preferably between 0.85 and 0.75.

The second end 20 of the tube 11 has a substantially circular shape and sizes mating with the spirometric instrument to which it is to be associated.

Merely by way of example, the internal diameter of the second end 20 is greater than or equal to 20 mm, in this case about 30 mm, and the tube 11 has a length greater than 50 mm, in this case about 70 mm.

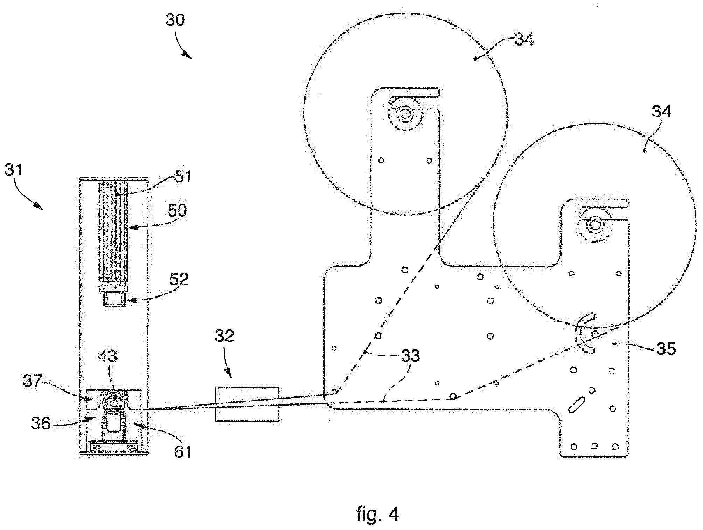

With reference to figs. 4-6, we shall now describe an apparatus 30 used to make a mouthpiece 10 of the type described above.

The apparatus 30 comprises an operating station 31 in which the operations to make the filtering element 14 are carried out, and also those to assemble the filtering element 14 on the tube 11.

The operating station 31 is served by a drawing unit 32 which draws layers 33 of filtering fabric which will define respectively the filtering fabric 15 and 16 of the filtering element 14.

The layers 33 are wound on support reels 34 mounted on a frame 35.

In the form of embodiment shown in fig. 4, two support reels 34 are mounted on the frame 35; two layers 33 to be sent to the operating station 31 are unwound from the reels 34 by the drawing unit 32.

The operating station 31 (fig. 4) is provided with a molding device 61 to make the filtering element 14.

In particular, the molding device 61 comprises a first molding element 36, or matrix, and a second molding element 37, or counter-matrix, configured to confer the shape and make the filtering element 14 by means of the layers 33.

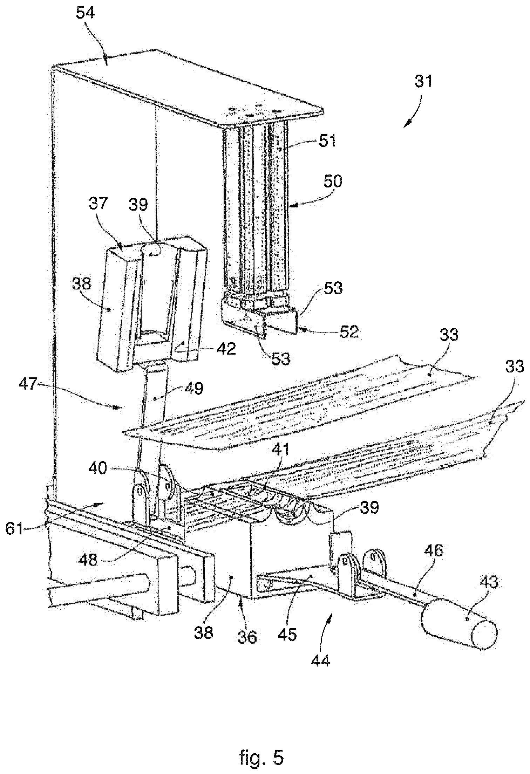

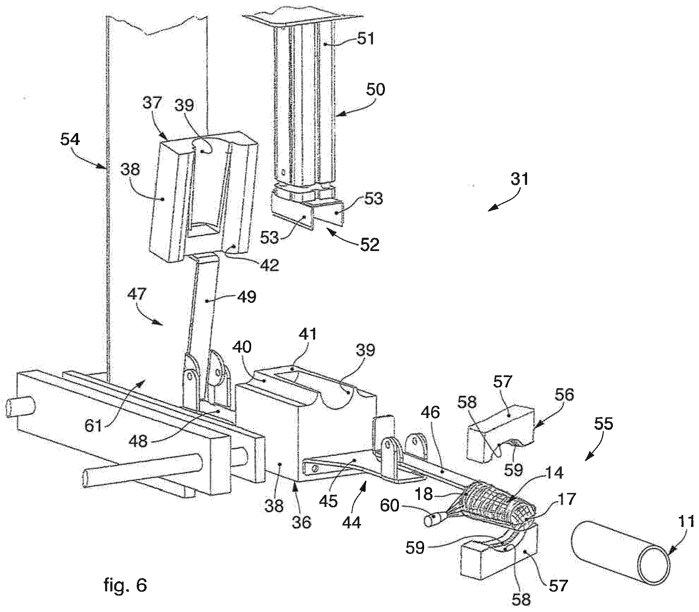

More specifically, both the matrix 36 and the counter-matrix 37 comprise a prismatic body 38 (figs. 5 and 6) in which respective cavities 39 are made, mating in shape with the external surface of the filtering element 14 to be obtained. In this way, when the cavities 39 of the matrix 36 and the counter-matrix 37 are brought close to each other, they define the external shape of the filtering element 14.

In particular, the cavity 39 of the matrix 36 is defined by a protruding portion 40 that comprises protruding edges 41 which cooperate during use with the counter-matrix 37.

The cavity 39 of the counter-matrix 37 is provided in a shaped seating 42 with shape and sizes mating with those of the protruding edges 41.

The counter-matrix 37 is connected to the matrix 36 by means of an articulated mechanism 47 that allows the correct positioning of the shaped seating 42 of the counter-matrix 37 in correspondence with the protruding edges 41 of the matrix 36.

The articulated mechanism 47 in this case comprises a bar 48 attached to the matrix 36, and an arm 49 pivoted with a first end to the bar 48 and having a second end, opposite the first, on which the counter-matrix 37 is attached.

Actuation means, not shown in the drawings, are provided to position the counter-matrix 37 on the matrix 36. In particular, the actuation means can be provided to articulate the arm 49 automatically to the bar 48.

The molding device 61 also comprises a core 43 (fig. 5) configured to be inserted between the cavities 39 of the matrix 36 and counter-matrix 37 and define, during use, the concavity of the filtering element 14.

The core 43 is shaped mating with the shape of the filtering element 14 to be obtained, in this case having a substantially truncated cone shape. In some forms of embodiment, the core 43 has its larger base substantially elliptical, ovoidal or circular and sizes mating with those of the first end 19 of the tube 11.

According to fig. 5, the core 43 is attached to a lever mechanism 44 that provides to position precisely the core 43 inside the cavities 39.

In particular, the lever mechanism 44 comprises a bracket 45 attached to the matrix 36, and a rod 46 hinged with one end to the bracket 45. The core 43 is attached in the other end of the rod 46.

The rotation of the rod 46 around the hinging point with the bracket 45 allows to position the core 43 in the cavity 39 of the matrix 36.

Actuation means, not shown in the drawings, can be provided to position the core 43 in the cavity 39 of the matrix 36 at the desired times. The actuation means can be provided to articulate the rod 46 with respect to the bracket 45.

The operating station 31 also comprises a punching device 50 that provides to cut and couple the two layers 33 of filtering fabric when they are disposed between the matrix 36 and counter-matrix 37.

The punching device 50 comprises an actuator 51 with which a tool 52 is associated to perform the punching operations.

The tool 52 is provided with two blades 53 disposed inclined convergent and distanced by the distance of the protruding edges 41. The blades 53 are configured to cut the layers 33 of filtering fabric in order to make the filtering element 14, as will be described hereafter.

It may be provided that the layers 33 of fabric are connected to each other by means of a gluing operation, heat welding, ultrasound or other connection techniques to define the sac-shaped filtering element 14.

The tool 52 can be the thermal type and/or ultrasound type, and provides both to cut and to join the layers 33 to define the filtering fabrics 15 and 16.

If the tool 52 is the thermal type, the blades 53 provide both to cut the layers 33 and also to perform a welding thereof.

A filtering element 14 is therefore obtained with a conical-trapezoidal shape, like a goose bill.

In fig. 5, the punching device 50 is mounted on a support arm 54 provided to support the actuator 51 and to dispose the tool 52 facing the matrix 36.

The operating station 31 is also provided with a connection device 55 (fig. 6) to connect the filtering element 14 to the tube 11.

The connection device 55 can comprise a vise 56 provided with two semi-shells 57 each having a semi-elliptical concavity. When the semi-shells 57 are disposed in contact with each other, the concavities 58 define an elliptical aperture mating with the shape and sizes that the first end 19 of the tube 11 will have to assume once the mouthpiece 10 is obtained in its final form.

Some forms of embodiment of the apparatus 30 provide that an ultrasound device 59 is also associated with the vise 56, and provides to reciprocally connect the filtering element 14 and the tube 11, as will be described hereafter.

The ultrasound device 59 can be provided inside the concavity 58 of the semi-shells 57 of the vise 56. It may be provided that the ultrasound device 59 is a separate and independent component from the vise 56.

Instead of providing to use the ultrasound device 59 to achieve the connection between filtering element 14 and tube 11, it is also possible to use a heat device.

The apparatus 30 can also comprise a spraying device 60 that provides to spray glue in correspondence with the end edge 18 of the filtering element 14. In this way a subsequent insertion of the filtering element 14 into the tube 11 allows to connect the two components to each other and to obtain the mouthpiece 10.

Merely by way of example, the spraying device 60 provides to spray the glue or to dispense it on an external circular portion of the filtering element, at a height comprised between 2 mm and 3 mm. The glue is chosen so as to meet the requirements of biocompatibility required for using the mouthpiece 10.

The apparatus 30 can be served by a machine for dispensing tubes 11, of a substantially known type, which provides to make the tubes 11 available to the operating station 31.

Merely by way of example, the dispensing machine comprises a vibrating drum to align and position the tubes 11 on a transfer unit which provides to position the tubes 11 in the desired position of the operating station 31.

Downstream of the apparatus 30 another machine may be provided for packaging each individual mouthpiece 10 obtained.

The method for making the mouthpiece 10 with the apparatus 30 as described above provides to unwind the layers 33 from the support reels 34 using the drawing unit 32, to make them available to the operating station 31.

In particular, one of the layers 33 is positioned above the matrix 36 and the core 43 is positioned in the cavity 39 of the matrix 36 so that the layer is comprised between the matrix 36 and the core 43.

Then the other layer 33 is positioned above the core 43. The counter-matrix 37 is positioned in contact with the matrix 36, closing both the core 43 and the two layers 33 between the cavities 39 of the matrix 36 and counter-matrix 37. The matrix 36 and the counter-matrix 37 confer on the layers 33 the shape that the filtering element 14 has to have.

Subsequently, the counter-matrix 37 is lifted from the matrix 36 to allow the subsequent actuation of the punching device 50.

In particular, the actuator 51 is actuated to take the tool 52 into contact with the layers 33 that are disposed on the matrix 36 and the core 43.

The tool 52 provides to cut both the layers 33 to define the shape of the filtering fabrics 15 and 16 and to weld them along the joining line 17, obtaining the filtering element 14 shaped mating with the shape of the core 43.

Subsequently, the core 43 in which the filtering element 14 is inserted is rotated by the lever mechanism 44, to dispose the core 43 in the operating position necessary to perform the connection of the filtering element 14 and the tube 11.

In particular, the core 43 with the filtering element 14 is disposed in proximity to the connection device 55.

In this condition it is provided to spray the glue on an external surface portion of the filtering element 14 using the spraying device 60, and to subsequently position the tube 11 on the core 43 so that the filtering element 14 is disposed completely inside the tube 11.

In this condition, the vise 56 is driven so that the semi-shells 57 act against the external surface 13 of the tube 11.

The action of the vise 56 not only makes the tube 11 and the filtering element 14 solid with each other due to the action of the previously distributed glue, but also allows to confer on the first end 19 of the tube 11 an anatomical elliptical shape so that the patient can easily put it in his/her mouth.

In order to speed up the operations of making the tube 11 and filtering element 14 solid, the vise 56 can be provided with heating elements.

Furthermore, in combination with or as an alternative to spraying with glue, it may be provided that making the tube 11 and filtering element 14 solid is achieved only by heat effect or ultrasound, or with the aid respectively of a heat device (not shown in the drawings) or the ultrasound device 59 associated with the vise 56, or independent thereof.

The apparatus 30 for making the mouthpiece 10 can also comprise a unit for making the cardboard tube 11.

In particular, the unit for making the cardboard tube 11 may comprise a mandrel, substantially cylindrical in shape, on which one or more strips of paper are wound, which are reciprocally coupled by gluing to define the tube. During winding, it may be provided to superimpose several layers of strips of paper to define the thickness of the tube. Cutting members may also be associated with the mandrel, configured to cut the tube axially and define its length.

## Claims

1. Mouthpiece for breathing apparatuses comprising a tubular body (11) provided with an internal surface (12), comprising a sac-shaped filtering element (14) attached peripherally with one open end edge (18) to said internal surface (12) of the tubular body (11) and completely contained inside it, **characterized in that** said tubular body (11) being made of cardboard, and said sac-shaped filtering element (14) comprising at least a filtering fabric consisting of a plurality of layers of filaments.

2. Mouthpiece as in claim 1, **characterized in that** said end edge (18) is attached in a permanent manner to said tubular body (11) by heat action, gluing, ultrasound or a combination thereof.

3. Mouthpiece as in claim 1 or 2, **characterized in that** the filtering element (14) is attached in proximity to a first end (19) of the tubular body (11) and has a peripheral development equal to the peripheral development of the internal surface (12) of said tube (11).

4. Mouthpiece as in claim 3, **characterized in that** the filtering element (14) is disposed with its convex portion facing toward a second end (20) of the tubular body (11) opposite the first end (19) and extends axially for at least 30% of the axial development of the tubular body (11).

5. Mouthpiece as in claim 3 or 4, **characterized in that** the first end (19) of the tubular body (11) has an oval shape.

6. Mouthpiece as in any claim hereinbefore, **characterized in that** the filtering element (14) comprises a plurality of filtering fabrics (15, 16) coupled to each other along at least a joining line (17).

7. Method to make a mouthpiece (10) for breathing apparatuses, comprising at least a step of making a tubular body (11) provided with an internal surface (12), comprising a step of making a sac-shaped filtering element (14) and a step of attaching peripherally an open end edge (18) of the filtering element (14) to the internal surface (12) of the tubular body (11); **characterized in that** said tubular body (11) is made of cardboard and **in that** said sac-shaped filtering element (14) comprises at least a filtering fabric consisting of a plurality of layers of filaments.

8. Method as in claim 7, **characterized in that** said step of making the filtering element (14) provides to couple at least two filtering fabrics (15, 16) together along a joining line (17).

9. Method as in claim 7 or 8, **characterized in that** during said attachment step it is provided to deform a first end (19) of said tubular body (11) in order to give it an elliptical shape.

10. Method as in any claim from 7 to 9, **characterized in that** said attachment step is carried out by means at least of one of the techniques of gluing, thermal attachment or ultrasound.

## Patentansprüche

1. Mundstück für Atemvorrichtungen, das einen rohrförmigen Körper (11) umfasst, der mit einer Innenfläche (12) versehen ist, umfassend ein sackförmiges Filterelement (14), das peripher mit einer offenen Endkante (18) an der Innenfläche (12) des rohrförmigen Körpers (11) befestigt und vollständig in diesem enthalten ist,
**dadurch gekennzeichnet, dass** der rohrförmige Körper (11) aus Karton hergestellt ist und das sackförmige Filterelement (14) mindestens ein Filtergewebe umfasst, das aus einer Vielzahl von Filamentschichten besteht.

2. Mundstück nach Anspruch 1, **dadurch gekennzeichnet, dass** die Endkante (18) durch Wärmeeinwirkung, Kleben, Ultraschall oder eine Kombination davon dauerhaft an dem rohrförmigen Körper (11) befestigt ist.

3. Mundstück nach Anspruch 1 oder 2, **dadurch gekennzeichnet, dass** das Filterelement (14) in der Nähe eines ersten Endes (19) des rohrförmigen Körpers (11) befestigt ist und eine Umfangsausbildung aufweist, die der Umfangsausbildung der Innenfläche (12) des Rohres (11) entspricht.

4. Mundstück nach Anspruch 3, **dadurch gekennzeichnet, dass** das Filterelement (14) mit seinem konvexen Abschnitt einem zweiten Ende (20) des rohrförmigen Körpers (11) zugewandt gegenüber dem ersten Ende (19) angeordnet ist und sich axial für mindestens 30 % der axialen Ausbildung des rohrförmigen Körpers (11) erstreckt.

5. Mundstück nach Anspruch 3 oder 4, **dadurch gekennzeichnet, dass** das erste Ende (19) des rohrförmigen Körpers (11) eine ovale Form aufweist.

6. Mundstück nach einem der vorhergehenden Ansprüche, **dadurch gekennzeichnet, dass** das Filterelement (14) eine Vielzahl von Filtergeweben (15, 16) umfasst, die entlang von mindestens einer Verbindungslinie (17) miteinander gekoppelt sind.

7. Verfahren zur Herstellung eines Mundstücks (10) für Atemvorrichtungen, umfassend mindestens einen Schritt zur Herstellung eines rohrförmigen Körpers (11), der mit einer Innenfläche (12) versehen ist, umfassend einen Schritt zur Herstellung eines sackförmigen Filterelements (14) und einen Schritt zur peripheren Befestigung einer offenen Endkante (18) des Filterelements (14) an der Innenfläche (12) des rohrförmigen Körpers (11); **dadurch gekennzeichnet, dass** der rohrförmige Körper (11) aus Karton hergestellt ist und dass das sackförmige Filterelement (14) mindestens ein Filtergewebe umfasst, das aus einer Vielzahl von Filamentschichten besteht.

8. Verfahren nach Anspruch 7, **dadurch gekennzeichnet, dass** der Schritt zum Herstellen des Filterelements (14) das Zusammenkoppeln von mindestens zwei Filtergeweben (15, 16) entlang einer Verbindungslinie (17) vorsieht.

9. Verfahren nach Anspruch 7 oder 8, **dadurch gekennzeichnet, dass** während des Befestigungsschritts ein Verformen eines ersten Endes (19) des rohrförmigen Körpers (11) vorgesehen ist, um ihm eine elliptische Form zu verleihen.

10. Verfahren nach einem der Ansprüche 7 bis 9, **dadurch gekennzeichnet, dass** der Befestigungsschritt mit mindestens einer der Techniken des Klebens, der thermischen Befestigung oder des Ultraschalls durchgeführt wird.

## Revendications

1. Embout buccal pour des appareils respiratoires comprenant un corps tubulaire (11) présentant une surface interne (12), comprenant un élément filtrant (14) en forme de sac fixé de façon périphérique, par un bord d'extrémité ouvert (18), à ladite surface interne (12) du corps tubulaire (11) et entièrement contenu à l'intérieur de celui-ci, **caractérisé en ce que** ledit corps tubulaire (11) est fait de carton, et ledit élément filtrant (14) en forme de sac comprenant au moins un tissu filtrant constitué d'une pluralité de couches de fibres.

2. Embout buccal selon la revendication 1, **caractérisé en ce que** ledit bord d'extrémité (18) est fixé de manière permanente audit corps tubulaire (11) par action thermique, collage, ultrasons ou une combinaison de ceux-ci.

3. Embout buccal selon la revendication 1 ou la revendication 2, **caractérisé en ce que** l'élément filtrant (14) est fixé à proximité d'une première extrémité (19) du corps tubulaire (11) et présente un développement périphérique égal au développement périphérique de la surface interne (12) dudit tube (11).

4. Embout buccal selon la revendication 3, **caractérisé en ce que** l'élément filtrant (14) est disposé avec sa partie convexe tournée vers une deuxième extrémité (20) du corps tubulaire (11) opposée à la première extrémité (19) et s'étend axialement sur au moins 30% du développement axial du corps tubulaire (11).

5. Embout buccal selon la revendication 3 ou la revendication 4, **caractérisé en ce que** la première extrémité (19) du corps tubulaire (11) a une forme ovale.

6. Embout buccal selon une quelconque des revendications ci-dessus, **caractérisé en ce que** l'élément filtrant (14) comprend une pluralité de tissus filtrants (15, 16) reliés les uns aux autres le long d'au moins une ligne de jonction (17).

7. Procédé de fabrication d'un embout buccal (10) pour appareils respiratoires, comprenant au moins une étape de fabrication d'un corps tubulaire (11) présentant une surface interne (12), comprenant une étape de fabrication d'un élément filtrant (14) en forme de sac et une étape de fixation périphérique d'un bord d'extrémité ouvert (18) de l'élément filtrant (14) sur la surface interne (12) du corps tubulaire (11) ; **caractérisé en ce que** ledit corps tubulaire (11) est en carton et **en ce que** ledit élément filtrant (14) en forme de sac comprend au moins un tissu filtrant constitué d'une pluralité de couches de filaments.

8. Procédé selon la revendication 7, **caractérisé en ce que** ladite étape de fabrication de l'élément filtrant (14) prévoit le fait de relier au moins deux tissus filtrants (15, 16) le long d'une ligne de jonction (17).

9. Procédé selon la revendication 7 ou la revendication 8, **caractérisé en ce que** pendant ladite étape de fixation, il est prévu de déformer une première extrémité (19) dudit corps tubulaire (11) pour lui donner une forme elliptique.

10. Procédé selon l'une quelconque des revendications 7 à 9, **caractérisé en ce que** ladite étape de fixation est mise en oeuvre au moyen d'au moins de l'une des techniques suivante : collage, fixation thermique ou ultrasons.
